# EUROPEAN PATENT SPECIFICATION

(11) **EP 0 582 368 B1**
(45) Date of publication and mention of the grant of the patent: **24.01.2001**
(21) Application number: 93304102.2
(22) Date of filing: 26.05.1993
(51) Int. Cl.: C07D 495/04, A61K 31/55

(54) **Thienobenzodiazepine derivatives for treatment of CNS disorders**
Thienobenzodiazepinderivate zur Behandlung von Störungen des Zentralnervensystems
Dérivés de thienobenzodiazépine pour le traitement des troubles du système nerveux central

(30) Priority: 29.05.1992 GB 9211379; 30.04.1993 GB 9309025
(43) Date of publication of application: 09.02.1994
(73) Proprietor: ELI LILLY AND COMPANY LIMITED, Basingstoke, Hants RG21 6XA (GB)
(72) Inventor: Fairhurst, John, Basingstoke, Hampshire RG22 5HA (GB); Hotten, Terrence Michael, Farnborough, Hampshire GU14 8PL (GB); Tupper, David Edward, Reading, Berkshire RG6 3AT (GB)
(74) Representative: Hudson, Christopher Mark

(56) References cited:
- EP-A- 0 454 436
- FR-A- 2 325 381
- JOURNAL OF MEDICINAL CHEMISTRY. vol. 23, no. 8, 1980, WASHINGTON US pages 878 - 884 J. K. CHAKRABARTI ET AL '4-Piperazinyl-10H -thieno(2,3-b)(1,5)benzodiazepines as potential neuroleptics'
- CHEMICAL ABSTRACTS, vol. 80, no. 1, 7 January 1974, Columbus, Ohio, US; abstract no. 3562x, 'Diazepine and thiazepine derivatives' page 314 ;

## Description

This invention relates to novel organic compounds and their use as pharmaceuticals.

Certain dibenzazepines are disclosed in French Patent 2 325 381 and Chem. Abs. 80 (1974), 3562x.

Similar thienobenzodiazepine compounds useful in the treatment of disorders of the central nervous system are described in British Patent 1 533 235 and European Patent Publication 0 454 436, and in J. Med. Chem. 23 (1980), 878-84.

The compounds of the present invention have the following formula 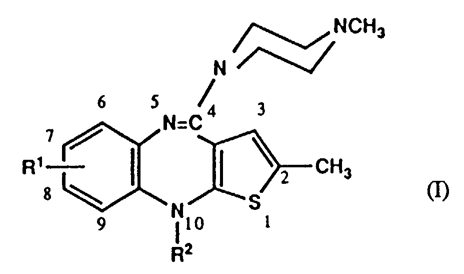 in which R¹ is hydrogen and R² is C₃ or C₄ alkyl; or a salt thereof.

These compounds are active in experimental screens for testing activity on the central nervous system, and the results indicate their usefulness in the treatment of a wide range of disorders of the central nervous system.

In the above formula R¹ can be halo and the halo substituent is preferably fluoro or chloro. The halo atom is preferably attached at the 7-position. When R² is C₁₋₁₀ alkyl, it can be straight or branched chain, such as, for example, methyl, ethyl, propyl, isopropyl, butyl, isobutyl, pentyl and octyl. A C₂₋₁₀ alkenyl group includes, for example, vinyl, prop-2-enyl, but-3-enyl, pent-4-enyl and oct-7-enyl. A C₂₋₁₀ alkynyl group includes, for example, prop-2-ynyl, but-3-ynyl, pent-4-ynyl and oct-7-ynyl. A C₃₋₆ cycloalkyl-C₁₋₄ alkyl group is a cycloalkyl group such as, for example, cyclopropyl, cyclobutyl, cyclopentyl or cyclohexyl, attached to C₁₋₄ alkyl, and a preferred example is cyclopropylmethyl. The C₃₋₆ cycloalkyl group can be substituted with 1 to 3 C₁₋₄ alkyl substituents, especially methyl. An optionally substituted phenyl group is phenyl or phenyl substituted with one or more, preferably one to three substituents selected from, for example, C₁₋₄ alkyl, especially methyl, C₁₋₄ alkoxy, especially methoxy or ethoxy, hydroxy, nitro, cyano, halo, especially chloro or fluoro, trihalomethyl, especially trifluoromethyl, carboxy or C₁₋₄ alkoxy-carbonyl. A preferred example of phenyl-C₁₋₄ alkyl is benzyl.

A particular group of compounds according to formula (I) is one in which R¹ is hydrogen or halo, and R² is C₁₋₄ alkyl, C₂₋₄ alkenyl, C₂₋₄ alkynyl, C₃₋₆ cycloalkyl-C₁₋₄ alkyl or optionally substituted phenyl-C₁₋₄ alkyl.

A further and preferred group of compounds according to formula (I) is one in which R¹ is hydrogen or halo, and R² is C₃₋₁₀ alkyl, C₃₋₁₀ alkenyl or C₃₋₁₀ alkynyl. Preferably R¹ is hydrogen, and preferably R² is C₃₋₁₀ alkyl, especially C₃₋₈ alkyl (more especially C₃ or C₄ alkyl and particularly propyl and isopropyl).

It will be understood that salts of the compounds of the invention can be prepared and such salts are included in the invention. They can be any of the well known acid addition salts. Preferably the salts are pharmaceutically acceptable, nontoxic addition salts with suitable acids, such as those with inorganic acids, for example hydrochloric, hydrobromic, nitric, sulphuric or phosphoric acids, or with organic acids, such as organic carboxylic acids, for example, glycollic, maleic, hydroxymaleic, fumaric, malic, tartaric, citric, salicylic, o-acetoxybenzoic, or organic sulphonic, 2-hydroxyethane sulphonic, toluene-p-sulphonic, or naphthalene-2-sulphonic acid.

If a phenyl nucleus on an R² substituent bears an acid function, base salts can be derived, for example, from ammonium hydroxide and alkali and alkaline earth metal hydroxides, carbonates and bicarbonates, as well as salts derived from aliphatic and aromatic amines, aliphatic diamines and hydroxy alkylamines. Bases especially useful in the preparation of such salts include ammonium hydroxide, potassium carbonate, sodium bicarbonate, lithium hydroxide, calcium hydroxide, methylamine, diethylamine, ethylene diamine, cyclohexylamine and ethanolamine. The potassium, sodium and lithium salt forms are preferred.

In addition to pharmaceutically-acceptable salts, other salts are included in the invention. They may serve as intermediates in the purification of compounds or in the preparation of other, for example pharmaceutically-acceptable, salts, or are useful for identification, characterisation or purification.

The invention also includes a process for preparing compounds of formula (I), which comprises alkylating the anion (at the 10-position) of a compound of the formula: 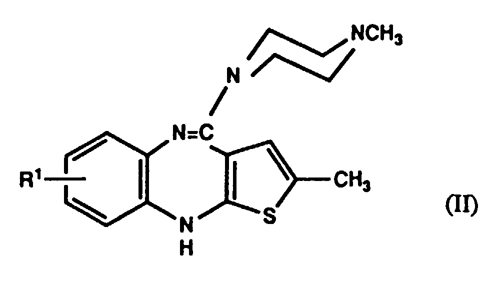 The reaction can be carried out according to well-known procedures using conventional alkylating agents of the formula R²X where X is a leaving group such as, for example, halo, especially chloro or bromo or C₁₋₄ alkylsulphonate, and a suitable base such as n-butyl lithium or sodium hydride. Preferably a reaction temperature of from -70° C. to +80° C., and an organic solvent such as, for example, tetrahydrofuran or dimethylformamide, are employed.

Alkylating agents of formula R²X are well-known and compounds of formula (II) can be made according to methods described in British Patent 1 533 235 and European Patent Publication 0 454 436.

As mentioned above, the compounds of the invention have useful central nervous system activity. This activity is indicated by models using well-established procedures. For example, the compounds are active in vitro binding assays designed to measure the degree of binding to neuronal receptors. The compounds are active at the dopamine D-1 receptor as indicated by an IC₅₀ of less than 1 µM in the ³H-SCH23390 (Billard, W. et al. Life Sciences 35, 1885 (1984)) binding assay. The compounds also have an antimuscarinic-anticholinergic activity.

Furthermore, the compounds are active at serotonin receptors. For example, some of the compounds, those in which R¹ is hydrogen and R² is C₃₋₈ alkyl, (especially C₃ and C₄ alkyl, and particularly propyl and isopropyl) show a high level of activity on 5-HT₃ receptors, as measured in the test described by Wong D. T. et al., European Journal of Pharmacology 166 (1989) 107-110. They also possess a degree of selectivity for these receptors over 5-HT₁ and 5-HT₂ receptors.

The compounds are also active in standard in vivo tests predictive of antipsychotic activity. They antagonised apomorphine-induced climbing behaviour in mice (Moore N. A. et al. Psychopharmacology 94 (2), 263-266 (1988), and 96, 539 (1988). The compounds also inhibit a conditioned avoidance response in rats.

Such tests indicate that the compounds are a potential neuroleptic with relaxant, anxiolytic or anti-emetic properties, and are useful in treating psychotic conditions such as schizophrenia, schizophreniform diseases and acute mania. At lower doses the compounds are indicated for use in the treatment of mild anxiety states. Furthermore, preferred compounds which have high levels of activity at the 5-HT₃ receptors, are also indicated for use in treating depression, memory deficit such as abnormal loss of memory, migraine, pain, and drug abuse, for example the undesired consumption of drugs such as alcohol, morphine, nicotine or haloperidol. They are also of potential use in treating anxiety disorders such as major anxiety and panic disorder.

The compounds of the invention are effective over a wide dosage range, the actual dose administered being dependent on the condition being treated. For example, in the treatment of adult humans, dosages of from 0.05 to 30 mg, preferably from 0.1 to 20 mg, per day may be used. A once-a-day dosage is normally sufficient, although divided doses may be administered. For treatment of psychotic disorders a dose range of from 2 to 15 mg, preferably 2.5 to 10 mg per day is suitable, whereas for mild anxiety states a lower dosage range, such as from 0.1 to 5 mg, preferably 0.5 to 1 mg, may be more appropriate. In choosing a suitable regimen for patients suffering from psychotic illness it may frequently be necessary to begin with a dosage of from 2 to 15 mg per day and when the illness is under control to reduce to a dosage as low as from 0.5 to 1 mg per day.

The compounds of the invention may be administered orally or by injection and, for this purpose, it is usually employed in the form of a pharmaceutical composition.

Accordingly the invention includes a pharmaceutical composition comprising as active ingredient a compound of formula (I) or a pharmaceutically-acceptable acid addition salt thereof, associated with a pharmaceutical-acceptable carrier. In making the compositions of the invention conventional techniques for the preparation of pharmaceutical compositions may be used. For example, the active ingredient will usually be mixed with a carrier, or diluted by a carrier, or enclosed within a carrier which may be in the form of a capsule, sachet, paper or other container. When the carrier serves as a diluent, it may be solid, semi-solid or liquid material which acts as a vehicle, excipient or medium for the active ingredient. The active ingredient can be adsorbed on a granular solid container, for example in a sachet. Some examples of suitable carriers are lactose, dextrose, sucrose, sorbitol, mannitol, starches, gum acacia, calcium phosphate, alginates, tragacanth, gelatin, syrup, methyl cellulose, methyl- and propyl-hydroxy-benzoate, talc, magnesium stearate or mineral oil. The compositions of the invention may, if desired, be formulated so as to provide quick, sustained or delayed release of the active ingredient after administration to the patient.

Depending on the method of administration, the compositions may be formulated as tablets, capsules, injection solutions for parenteral use, suspensions or elixirs for oral use or suppositories. Preferably the compositions are formulated in a dosage unit form, each dosage containing from 0.1 to 20 mg, more usually 0.5 to 10 mg, of the active ingredient.

A preferred formulation of the invention is a capsule or tablet comprising 0.1 to 20 mg or 0.5 to 10 mg of active ingredient together with a pharmaceutically-acceptable carrier therefor. A further preferred formulation is an injection which in unit dosage form comprises 0.1 to 20 mg or 0.5 to 10 mg of active ingredient together with a pharmaceutically-acceptable diluent therefor. A type of injection formulation that is also desirable is a sustained release formulation for intramuscular injection, in which case, a unit dose may preferably contain up to 100 mg.

The invention is illustrated by the following Examples.

### EXAMPLE 1

### 2,10-Dimethyl-4-(4-methyl-1-piperazinyl)-10H-thieno[2,3-b][1,5]benzodiazepine

A solution of 2-methyl-4-(4-methyl-1-piperazinyl)-10H-thieno[2,3-b][1,5]benzodiazepine (3.1 g) (European Patent Publication 0 454 436) in dry tetrahydrofuran (distilled from sodium/benzophenone) was stirred and cooled to -70° C. Tetramethylethylenediamine (1.16 g) was added followed by n-butyllithium (6.25 ml, 1.6M solution), keeping the temperature below-60° C. The deep red solution was stirred for 15 minutes and then methyl iodide (1.42 g) was added. The reaction was allowed to attain room temperature, during which time the red colour discharged. Water was added and the product extracted with ethyl acetate. The solvent was washed with water, dried and evaporated to dryness under reduced pressure. The product was purified by chromatography on Florisil using ethyl acetate as eluent and crystallised from acetonitrile to give the title compound, m.p. 126-128° C.

Similarly prepared from the same starting material were:

10-Ethyl-2-methyl-4-(4-methyl-1-piperazinyl)-10H-thieno[2,3-b][1,5]benzodiazepine m.p. 125-127° C.

2-Methyl-4-(4-methyl-1-piperazinyl)-10-(prop-2-enyl)-10H-thieno[2,3-b][1,5]benzodiazepine m.p. 110-112° C.

2-Methyl-4-(4-methyl-1-piperazinyl)-10-(prop-2-ynyl)-10H-thieno[2,3-b][1,5]benzodiazepine m.p. 155-156° C.

10-Benzyl-2-methyl-4-(4-methyl-1-piperazinyl)-10H-thieno[2,3-b][1,5]benzodiazepine m.p. 188-190° C. (as hydrochloride salt).

10-Butyl-2-methyl-4-(4-methyl-1-piperazinyl)-10H-thieno[2,3-b][1,5]benzodiazepine m.p. 235-237° C. (as hydrochloride salt).

2-Methyl-4-(4-methyl-1-piperazinyl)-10-(2-methyl-propyl)-10H-thieno[2,3-b][1,5]benzodiazepine m.p. 238-240° C. (as hydrochloride salt).

2-Methyl-4-(4-methyl-1-piperazinyl)-10-pentyl-10H-thieno[2,3-b][1,5]benzodiazepine m.p. 210-212° C. (as hydrochloride salt).

2-Methyl-4-(4-methyl-1-piperazinyl)-10-octyl-10H-thieno[2,3-b][1,5]benzodiazepine m.p. 208-210° C. (as hydrochloride salt).

Similarly prepared from 7-fluoro-2-methyl-4-(4-methyl-1-piperazinyl)-10H-thieno[2,3-b][1,5]benzodiazepine (British Patent 1 533 235) were:

7-Fluoro-2,10-dimethyl-4-(4-methyl-1-piperazinyl)-10H-thieno[2,3-b][1,5]benzodiazepine m.p. 145-147° C.

10-Ethyl-7-fluoro-2-methyl-4-(4-methyl-l-piperazinyl)-10H-thieno[2,3-b][1,5]benzodiazepine m.p. 128-130° C.

7-Fluoro-2-methyl-4-(4-methyl-1-piperazinyl)-10-propyl-10H-thieno[2,3-b][1,5]benzodiazepine m.p. 67-70° C.

10-Cyclopropylmethyl-7-fluoro-2-methyl-4-(4-methyl-1-piperazinyl)-10H-thieno[2,3-b][1,5]benzodiazepine m.p. 50-52°C.

7-Fluoro-2-methyl-4-(4-methyl-1-piperazinyl)-10-(prop-2-enyl)-10H-thieno[2,3-b][1,5]benzodiazepine m.p. 108-111° C.

7-Fluoro-2-methyl-4-(4-methyl-1-piperazinyl)-10-(prop-2-ynyl)-10H-thieno[2,3-b][1,5]benzodiazepine m.p. 50-55° C.

10-Benzyl-7-fluoro-2-methyl-4-(4-methyl-1-piperazinyl)-10H-thieno[2,3-b][1,5]benzodiazepine m.p. 65-67° C.

### EXAMPLE 2

2-Methyl-10-(1-methylethyl)-4-(4-methyl-1-piperazinyl)-10H-thieno[2,3-b][1,5]benzodiazepine.

To a solution of 2-methyl-4-(4-methyl-1-piperazinyl)-10H-thieno[2,3-b][1,5]benzodiazepine (3.12 g) in dry dimethylformamide (50 ml) was added sodium hydride (0.63 g, 50% dispersion). The reaction mixture was stirred for 30 minutes at room temperature, then 30 minutes at 60-70° C. The reaction was cooled to 40° C., isopropyl bromide (1.13 ml) added and left for one hour. Water was added and the reaction extracted with ethyl acetate, washed with water, dried and evaporated under reduced pressure. Chromatography using 2% methanol/dichloromethane and flash silica gave the title compound, m.p. 120-121° C.

Similarly prepared from the same starting material were:

2-Methyl-4-(4-methyl-1-piperazinyl)-10-propyl-10H-thieno[2,3-b][1,5]benzodiazepine m.p.122-123° C.

10-Cyclopropylmethyl-2-methyl-4-(4-methyl-1-piperazinyl)-10H-thieno[2,3-b][1,5]benzodiazepine m.p. 108-111° C.

The following formulations can be made using an active compound of the invention.

### EXAMPLE 3

A tablet formulation is made by granulating the active with appropriate diluent, lubricant, disintegrant and binder and compressing

| | |
|---|---|
| Compound of the invention | 5.0 mg |
| Magnesium stearate | 0.9 mg |
| Microcrystalline cellulose | 75.0 mg |
| Povidone | 15.0 mg |
| Starch, directly compressible | 204.1 mg |

### EXAMPLE 4

An aqueous injection of active is prepared as a freeze-dried plug, for reconstitution in a suitable, sterile diluent before use (to a total volume of 10 ml).

| | |
|---|---|
| Compound of the invention | 20.0 mg |
| Mannitol | 20.0 mg |
| N Hydrochloric acid and/or N sodium hydroxide to adjust pH to 5-5.5. | |

### EXAMPLE 5

A controlled release injection for intramuscular injection is formed from a sterile suspension of micronised active in an oleaginous vehicle.

| | |
|---|---|
| Compound of the invention | 65.0 mg |
| Aluminium stearate | 0.04 mg |
| Sesame oil | 2 ml |

### EXAMPLE 6

A formulation is prepared by blending the active with starch and silicone starch, and filling it into hard gelatine capsules.

| | Per 290 mg capsule |
|---|---|
| Compound of the invention | 2.5 mg |
| Starch flowable with 0.96% silicone 220 | 217.5 mg |
| Starch flowable | 70.0 mg |

## Claims

1. A compound of the formula 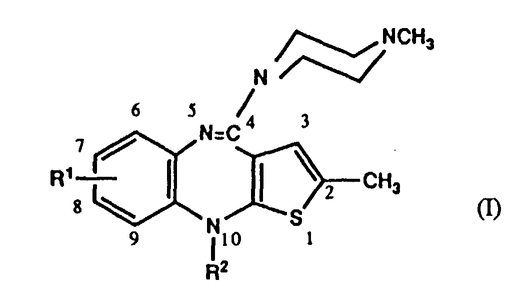 in which R¹ is hydrogen and R² is C₃ or C₄ alkyl; or a salt thereof.

2. A pharmaceutical formulation comprising a compound according to claim 1, or a pharmaceutically-acceptable salt thereof, together with a pharmaceutically-acceptable diluent or carrier therefor.

3. A compound according to claim 1, or a pharmaceutically-acceptable salt thereof, for use as a pharmaceutical.

4. A process for producing a compound according to claim 1, which comprises alkylating an anion of a compound of the formula 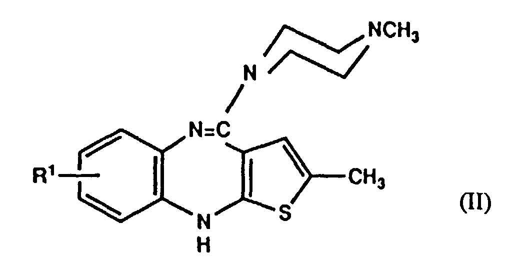 in which R¹ is hydrogen.

## Patentansprüche

1. Verbindung der Formel 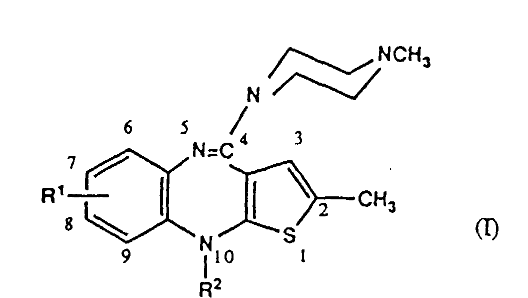 wobei R¹ Wasserstoff ist und R² C₃ oder C₄ Alkyl ist; oder ein Salz davon.

2. Pharmazeutische Formulierung, umfassend eine Verbindung nach Anspruch 1 oder ein pharmazeutisch verträgliches Salz davon, gemeinsam mit einem pharmazeutisch verträglichen Verdünner oder Träger dafür.

3. Verbindung nach Anspruch 1 oder pharmazeutisch verträgliches Salz davon zur Verwendung als Arzneimittel.

4. Verfahren zur Herstellung einer Verbindung nach Anspruch 1, welches Alkylieren eines Anions einer Verbindung der Formel 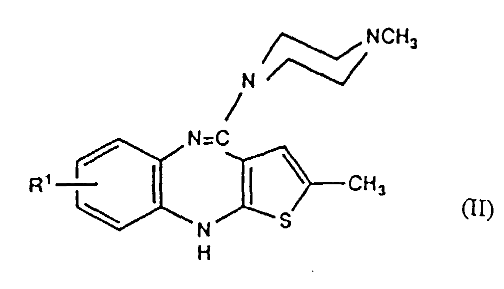 umfaßt, wobei R¹ Wasserstoff ist.

## Revendications

1. Composé de formule 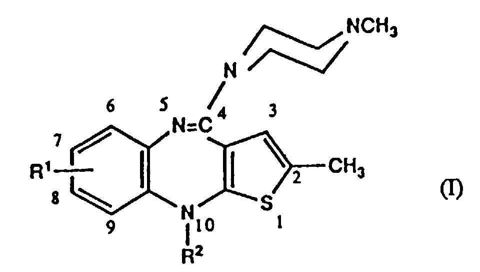 dans laquelle R¹ est un atome d'hydrogène et R² est un groupe alkyle en C₃ ou C₄ ; ou un de ses sels.

2. Formulation pharmaceutique comprenant un composé selon la revendication 1, ou un de ses sels pharmaceutiquement acceptables, conjointement avec un diluant ou support pour celui-ci pharmaceutiquement acceptable.

3. Composé selon la revendication 1, ou un de ses sels pharmaceutiquement acceptables, destiné à être utilisé comme produit pharmaceutique.

4. Procédé de production d'un composé selon la revendication 1, qui comprend l'alkylation d'un anion d'un composé de formule 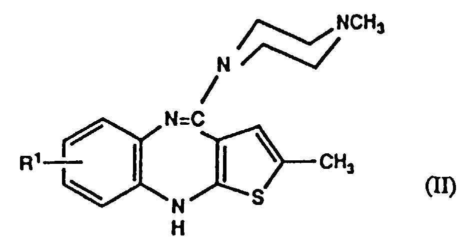 dans laquelle R¹ est un atome d'hydrogène.
